# EUROPEAN PATENT SPECIFICATION

(11) **EP 1 218 532 B1**
(45) Date of publication and mention of the grant of the patent: **26.12.2007**
(21) Application number: 00965134.0
(22) Date of filing: 19.09.2000
(51) Int. Cl.: C12Q 1/00

(54) **Method for monitoring an analyte using an electrochemical Biosensor which can be turned off by applying an electrical potential**
Methode zur Messung eines Analyten mit Hilfe eines elektrochemischen Biosensors, der durch Anlegen eines Potentials abgeschaltet werden kann
Méthode de détection d'un analyte utilisant un bio-capteur électrochimique qui peut être éteint par l'application d'une potentiel électrique

(30) Priority: 20.09.1999 US 154731 P
(43) Date of publication of application: 03.07.2002
(73) Proprietor: Roche Diagnostics Operations, Inc., Indianapolis, IN 46250-0416 (US); Roche Diagnostics GmbH, 68305 Mannheim (DE)
(72) Inventor: BUCK, Harvey, B., Indianapolis, IN 46256 (US); ESSENPREIS, Matthias, Fremont, CA 94536 (US)
(74) Representative: Jung, Michael
(86) International application number: PCT/US2000/025631
(87) International publication number: WO 2001/021827

(56) References cited:
- WO-A-93/06237
- WO-A-95/29199
- WO-A-97/00441
- WO-A-98/35225
- WO-A-98/58250
- US-A- 5 735 273

## Description

### Field of the Invention

This invention relates a method of using a sensor for detecting the presence or quantity of an analyte in a biological fluid. More particularly, this disclosure is directed to a sensor adapted particularly for indwelling or implanted use. Analyte concentrations are measured electrochemically in a contained detection retention volume of an analyte-permeable medium optionally separated from the biological fluid with a semi-permeable membrane.

### Background and Summary of the Invention

There has been a significant research and development effort directed to the development of electrochemical sensors capable of detecting the presence and/or quantity of biologically significant analytes. Many, if not most of such analyte sensing devices are in the form of a test strip comprising a test fluid containment space pretreated with an analyte-dependent detection composition and electrodes for contact with test fluid delivered into the test fluid containment space. Electrical conductors extend from the electrodes to an area on the test strip for connection to a hand held or table mounted preprogrammed sensor reading device. Typically a biological fluid is delivered to the sample fluid containment area or volume and the sensor reading device is programmed to apply a predetermined potential to the electrodes after a predetermined period of time following delivery of the fluid sample to the sample containment space. Current flow is then measured responsive to said applied potential to provide an indication of the presence and/or concentration of the target analyte.

Some of such electrochemical sensors are constructed to prevent direct contact of the sample fluid with the electrodes by covering the electrodes with a semipermeable membrane or gel matrix material, which is insoluble in the test medium and permeable at least to the analyte of interest when in contact with said test medium.

There is a continuing need for the development of commercially feasible, multi/continuous-use sensors for biologically significant analytes. In particular, there is need for development of biological sensors capable of being implanted or injected into a patient for periods ranging from several hours up to several weeks, months, or years and designed to provide accurate results without removal or recalibration to compensate for changes in diffusional properties of membrane components or for losses of enzyme activity and/or electron mediator elements. Such sensors would find application as components of artificial organs, for example an artificial pancreas, requiring continuous and/or regular monitoring of patient glucose levels. Such devices could also find use as reusable sensors for measuring analyte concentrations in bodily fluids *in vitro,* such as the analytical situations encountered by commercial labs performing analysis on patient fluid samples.

There are unique problems presented by the design and construction of biological sensors capable of repeated use *in vitro* and/or continuous use *in vivo.* Indeed, inherent in such functional requirements is the condition that the functional chemical component of the sensor be confined, i.e. not released from the sensor into the sample fluid during repeated and/or continuous use. The retention of the "active" chemical/electrochemical components of the biosensor can be accomplished by one of several techniques, alone or in combination. Thus the active components can be immobilized, for example, by covalent bonding to non-leachable components of the biosensor or by confining the biologically/electrically active components in a testing zone or volume by means of a membrane permeable at least by the analyte, but not by the contained, optionally covalently bound, enzymes, coenzymes, and/or electron mediators.

The implantable and/or reusable biosensors are designed to retain the active sensor-dependent chemical components, typically in a hydrophilic matrix in an analyte retention volume. The active electrochemical species that cooperate in the sensor responsive to an applied potential to provide a current flow signal proportionate to the concentration of analyte diffused into the retention volume can optionally be covalently bound to non-leachable components of the retention volume including, but not limited to, an electrode of an electrode system, a wall of the enclosure portion of the sensor for defining, at least in part, the retention volume, to microspheres or other microparticulate solids contained in the retention volume, to the retention volume contacting the side of a membrane, or to polymer components of the retention volume matrix. Alternatively the enzyme(s), the enzyme cofactor(s) and the electron mediator(s) can be selected to have a molecular weight sufficiently high to preclude any substantial diffusion of such components from the retention volume into the biological fluid being analyzed.

In one aspect of this disclosure the retention volume medium, alternatively denominated the "depletion volume medium" is in contact with the electrode system comprising an electrode capable of receiving electrons from or delivering electrons to the enzyme(s) via the electron mediator(s). Conductor elements extend from the electrode to a point on the device for allowing electrical communication of the electrode with a programmable controller. The controller can be programmed to apply a predetermined potential sequence to the electrode system including variable potential including either a mediator oxidizing potential or mediator reducing potential, variable pulse width and variable pulse intervals. The controller is also capable of sensing current flow responsive to applied potential(s) to the electrode system and comparing such data with control data previously obtained for said system to calculate and report analyte concentrations in the biological sample being analyzed and, optionally, to use such data to sense the performance status of the device and use such for modifying the then existing potential sequence protocol to optimize device function. Thus, for example, the sensor control can be modified periodically to adjust for differences in analyte diffusion efficiency across the membrane and/or changes in concentration of the active electron mediator and/or enzyme component(s) of the device without use of classical recalibration techniques.

In one embodiment of the disclosure the retention volume is defined or enclosed, at least in part, by an analyte-permeable membrane and the ratio of the retention volume to the surface area of the semipermeable membrane defining that volume, at least in part, is less than 2mm, more preferably less than 1mm. The low volume to surface area ratios are preferred in that they improve the rate of diffusional equilibrium between the fluid being tested and the retention volume medium, and thereby it works to minimize the refractory period (the recovery period) of the sensor.

In one preferred embodiment of the disclosure the enzyme component is selected so that it is substantially not capable of transferring electrons to or from any endogenous substance other than said analyte. Under such conditions the enzyme reaction responsible for providing a signal of analyte concentration cannot take place without a predetermined threshold potential being applied to the electrode system. The sensor can therefore be turned off to stop enzyme activity following a pulse of reducing potential to "deactivate" the mediator, and allow predictable concentration-gradient-based diffusion to work to rapidly "reset" the analyte concentration in the analyte detection/retention volume for the next programmed pulsed potential detection sequence.

In the present invention, which is specified in the appended claims, there is provided a method for monitoring analyte concentration using the sensor of this invention by contacting the sensor with the biological fluid being analyzed. Initially at predetermined intervals a potential is applied intermittently to the electrode system sufficient to oxidize the electron mediator in the retention volume, and the current flow through the electrode is sensed as a function of the duration of the applied potential. The applied mediator oxidizing potential is maintained at least for a period of time sufficient to determine the rate of change of current through the electrode as a function of duration of the applied potential. Values for the sensed current are correlated with values of current flow for known concentrations of the analyte. Alternatively the sensing protocol can comprise adjusting the potential to establish a predetermined current flow and thereafter sensing the rate of change of potential required to maintain said current flow for a predetermined time period.

In another embodiment the analyte concentration in a biological sample is measured as a function of the time dependent concentration of analyte in the retention volume following analyte depleting potential pulses. The rate of recovery concentration in the retention/depletion volume can be readily correlated with analyte concentration in the biological fluid contacting the sensor. The "diffusion status" of the membrane can be checked by a preprogrammed sequence from time-to-time during sensor use and numerical values associated with the sensed status can be used as input to modify the preprogrammed pulse sequence algorithms for subsequent sensor operation.

These and other features of the invention are described hereinbelow with reference to the drawings and the best mode known for carrying out the invention.

### Brief Description of the Drawings

Fig. 1 is a plan view of a sensor.
Fig. 2 is a cross-sectional view of the sensor along lines 2-2 of Fig. 1.
Fig. 3 illustrates a cross-sectional view similar to Fig. 2 but the electrode is of different construction.
Fig. 4 is similar to Fig. 2 illustrating a cross-sectional view of a sensor having a diffusion-limiting membrane with large pores.
Fig. 5 is similar to Fig. 4 illustrating a cross-sectional view of a sensor wherein diffusion into the retention volume is via pores on the periphery of an otherwise non-permeable membrane component.
Fig. 6 is a graphic representation of a pulse sequence in which two pulses of oxidative potential of different duration are applied to the sensor, interspersed with recovery intervals with reducing potential.
Fig. 7 is a graphic representation of a pulse sequence in which the duration of the intervals between the pulses is changed
Figs. 8-9 are graphic representations of measurement protocols, which combine changes in the pulse interval with changes in the pulse width.
Fig. 10 is similar to Fig. 1 and is a plan view of a sensor.
Fig. 11 is a graph showing the measured response for the Pyrrole-3-acetic acid/Mediator/glucose dehydrogenase (GDH) sensor.
Fig. 12 is a graph showing the measured response for the Pyrrole-3-carboxylic acid/mediator/GDH sensor.
Fig. 13 is a graph showing the response of the Pyrrole-3-acetic acid/Mediator/GDH and the Pyrrole-3-carboxylic acid/mediator/GDH sensors.

### Detailed Description of the invention

The electrochemical sensor of this disclosure is designed to provide signals indicative of analyte concentration in a biological fluid. The sensor comprises an electrode in contact with a low volume of a hydrophilic medium for retaining an amount of analyte proportionate to the concentration of analyte in a biological fluid in contact with the sensor. The medium is selected to allow facile analyte diffusion through said medium alone or upon hydration of said medium prior to or consequent to sensor use. The sensor comprises an enclosure for the volume of hydrophilic medium. The enclosure is formed to expose the hydrophilic medium to the biological fluid so that analyte in the biological fluid diffuses into the hydrophilic medium until the concentration of analyte in the medium is equivalent to the concentration of analyte in the biological fluid. The rate of analyte mass diffusion into the retention volume is dependent on the analyte concentration gradient. The concentration of analyte in the retention/depletion volume may be measured electrochemically by cooperation of an electron mediator and a redox enzyme specific for the analyte, each forming part of or being in contact with the hydrophilic medium.

The hydrophilic medium can have a water concentration level less than, equal to or greater than the water content of the biological fluid. Thus the components of the hydrophilic medium including the enzyme and electron mediator components and a hydrophilic polymer can be used in construction of the sensor in a substantially dehydrated state, ready for rehydration prior to use or upon contact of the sensor with a biological fluid. It is important for sensor function that the analyte of interest is readily diffusible through the hydrophilic medium to enable a substantially homogeneous concentration of the analyte in the analyte retention volume, and a concentration that closely corresponds to analyte concentration in the biological fluid in contact with the sensor.

The sensor is constructed to have an enclosure or compartment for holding the analyte retention volume of the hydrophilic medium. The enclosure compartment is formed to expose the hydrophilic medium to the biological fluid when the sensor is in use. In one embodiment, the enclosure compartment is defined at least in part by a wall comprising an area of an analyte permeable membrane having a first side in contact with the hydrophilic medium and an opposite side for contact with the biological fluid when the sensor is in use. The analyte of interest water, and other membrane permeable components of the biological fluid diffuses through the membrane and into the retention volume of hydrophilic medium until the concentration of analyte in the medium is proportionate to the concentration of analyte in the biological fluid contacting the analyte permeable membrane component of the sensor.

The sensor is constructed to provide an electrode in electrical contact with the hydrophilic medium. The electrode is typically formed of a conductive element such as carbon, silver, gold, platinum, palladium and the like and typically extends into or forms part of the walls of the container or chamber for the analyte retention volume of hydrophilic medium. In one embodiment the electrode is formed of platinum and the retention volume is defined as the space overlying the electrode. In another embodiment, the electrode can be formed of a graphite powder and the retention volume is defined by the intraparticulate spaces and, in at least one embodiment, an overlying analyte permeable membrane. In another embodiment the electrode is a component of an electrode system comprising a reference electrode and optionally an auxiliary electrode, which may be different or identical to the reference electrode. The electrode system can also comprise conductor elements for providing electrical communication between the electrode components of the system and a programmable controller to control the electrical potentials in said electrode system and to sense current flow through at least one of said electrodes responsive to said electrical potentials. Typically the programmable controller is constructed as a separate unit with electrical connectors adapted particularly for electrical communication between the controller and the electrode system of the sensor. The controller is typically a hand-held or table mounted unit capable of being reversibly connected to one or more biosensors and having data storage and data display elements.

The sensor for electrochemical analysis in accordance with this disclosure further comprises a redox enzyme and an electron mediator in contact with the hydrophilic medium. The enzyme is selected for its capacity to oxidize or reduce the analyte of interest. The enzyme is preferably selected as well for its lack of capacity for transferring electrons to or from substances other than said analyte that are capable of diffusing from the biological fluid into the analyte retention volume. Non-limiting examples of suitable enzymes include pyrroloquinoline quinone (PQQ) -dependent glucose dehydrogenase *(GDH)* (EC 1.1.99.17) or Hydroxybutyrate dehydrogenase (HBDH) (EC 1.1.1.30). Dehydrogenase enzymes for other diffusible analytes are known in the art and can be substituted dependent on the analyte of interest.

The electron mediator can be selected from any of a wide variety of electron mediators capable of facilitating transfer of electrons between the redox enzyme and a sensor electrode in contact with the medium containing or in contact with said enzyme and said mediator. A non-limiting example of a suitable mediator includes osmium (bis-bipyridyl) pyridinium chloride. It is appreciated, however, that a number of commercially available mediators, non-limiting examples of which are described in U.S. Patent No. 5,589,326, may be used. The enzyme and electron mediator can be entrapped in a polymer matrix on the electrode. Optionally, the enzyme and electron mediator can be selected to minimize their diffusion through an analyte permeable membrane during sensor use or can be covalently bound to the walls of the enclosure or to hydrophilic polymer components of the retention medium to minimize, if not prevent, their diffusion from the retention medium volume through the membrane and into the biological fluid during sensor use.

Any of a wide variety of hydrophilic polymers, typically having a molecular weight in excess of 5000 Daltons and having polyanionic, polycationic, or polyhydric functionality can be used as a carrier or carrier matrix forming part of the hydrophilic medium component of the sensor. Examples of such polymers include cellulosic polymers such as cellulose acetate, hydroxy ethyl cellulose, polyethylene glycols, synthetic or natural gums such as guar or xanthan gums, alginic acid, poly (meth) acrylic acids and copolymers of acrylic acids and acrylic esters, glycosaminoglycans, and the like which polymers can be used. Additionally, electrically polymerized matrices from monomers such as pyrrole-3-acetic acid and pyrrole-3-carboxylic acid can serve as the hydrophilic matrix and/or enzyme entrapping matrix.

Such hydrophilic polymers can be used alone or in combination to provide a hydrated or hydratable matrix through which the targeted analyte is readily diffusible. Further, such polyfunctional hydrophilic polymers can be used to "anchor" or otherwise impair the diffusion of the enzyme or electron mediator components of the medium to minimize loss of such components from the retention volume during sensor use.

Thus, art-recognized electron mediators having, for example hydroxy, carboxy or amino functionality for example ferrocene carboxylic acid, can be coupled using art-recognized ester-forming or amide-forming coupling methodologies to form the hydrophilic medium for use in preparation of the present sensors. Additional non-limiting examples of mediator compounds include osmium-containing redox mediators such as those described in U.S. Patent No. 5,589,326, and mediator compounds that can be tethered to a polymeric matrix, which include redox reversible imidazole-osmium complexes. Non-limiting examples of such complexes include osmium-bipyridyl conjugates such as bis(bipyridyl) imidazolyl haloosmium complexes characterized by fast mediation kinetics and low redox potential (+150 mV vs. Ag/AgCl). Another group of osmium containing mediators include tris(bipyridyl) osmium complex labeled electrochemically detectable conjugates. The redox enzyme and an electron mediator can thus be inherently non-diffusible or chemically coupled with the high molecular weight components of the medium to render the enzyme and electron mediator components substantially not capable of diffusing through the analyte permeable membrane during sensor use in contact with a biological fluid.

The enzyme component of the sensor is typically of sufficient molecular weight that diffusional loss of that component through the semipermeable membrane is marginal over the typical period of sensor use. Such enzymes can be incorporated into the hydrophilic medium during device construction, as, for example, an enzyme lyophilizate, formed by freeze drying a solution of enzyme in the presence of a hydrophilic monomer, for example, maltose or trehalose, or other enzyme stabilizing hydrophilic composition. The lyophilized enzyme can be retained in said medium during sensor manufacture and storage in a dehydrated state until rehydration prior to or during initial use of said sensor, thereby providing longer sensor shelf life.

Electron mediator components of the sensors of the present disclosure are not critical except for the fact that they should be selected or modified, for example by covalent bonding of polymer components of the hydrophilic matrix or the hydrophilic medium, to prevent or minimize diffusional loss of the electron mediator component from the analyte retention volume medium during the course of sensor use. The prior art is replete with reference to a wide variety of compounds including metal chelates and other metal complexes such as ferrocene, and more particularly carboxy ferrocene that can be readily coupled covalently to non-diffusible components of the hydrophilic medium.

The membrane components of the sensor constructs of this disclosure can be any biocompatible analyte permeable membrane, including for example cellulose acetate, polyurethane, and polycarbonate. Other polymeric biocompatible membranes suitable for use in biosensor construction are also well known in the art and any of such art-recognized analyte permeable membrane/membrane materials may be used in manufacture of the present sensors. Example of analyte permeable membranes, and as well electron mediators and redox enzymes are describe in U.S. Patent No. 5,264,105.

In one embodiment of the disclosure the hydrophilic matrix is bound on the surface of at least one of the electrodes of the electrode system. The hydrophilic matrix comprises an electron mediator covalently bound to a non-diffusible or poorly diffusible hydrophilic polymer component of the matrix. The redox enzyme is bound to a polymer component of the hydrophilic matrix as well. A sensor of the present disclosure preferably already includes the electron mediator and redox enzyme components, which are exposed to analyte present in the retention volume of the sensor. Initially at predetermined intervals a potential is applied intermittently to the electrode system sufficient to oxidize the electron mediator and the current flow through the electrode is sensed as a function of the duration of the applied potential. The applied mediator oxidizing potential is maintained at least for a period of time sufficient to determine the rate of change of current through the electrode as a function of duration of the applied potential. Values for the sensed current are correlated with values of current flow for known concentrations of analyte.

In another embodiment the hydrophilic medium comprises either a polymeric electron mediator or an electron mediator covalently bound to a non-diffusible or poorly diffusible hydrophilic polymer component of the medium. The redox enzyme is included as a stabilized lyophilizate or is covalently bound itself to a polymer component of the hydrophilic medium. The hydrophilic medium also comprises polyfunctional components that can be reacted with difunctional crosslinking agents contacted with the surface of said hydrophilic medium to form *in situ* an analyte permeable membrane on the surface of the hydrophilic medium. Thus for example a polyhydric polymer or a di- or trihydric, preferably high molecular weight, monomer component of the hydrophilic medium can be reacted, for example, with a polyisocyanate, for example a diisocyanate in the vapor phase, to form a polymer skin or membrane on the surface of the hydrophilic medium. The permeability of the membrane can be controlled by the length of exposure of the polyhydric medium surface to the multifunctional crosslinker. Thus, for example, 1,4-benzene diisocyanate can be vaporized in a chamber. Sensor constructs comprising the hydrophilic medium, preferably already including the electron mediator and redox enzyme components, having an exposed surface is introduced into the chamber for a period of time sufficient to form a biocompatible membrane on the surface of the hydrophilic medium to define in conjunction with other sensor components for example, a simple planar non-conductive substrate, the enclosure for the analyte retention volume component of the sensor.

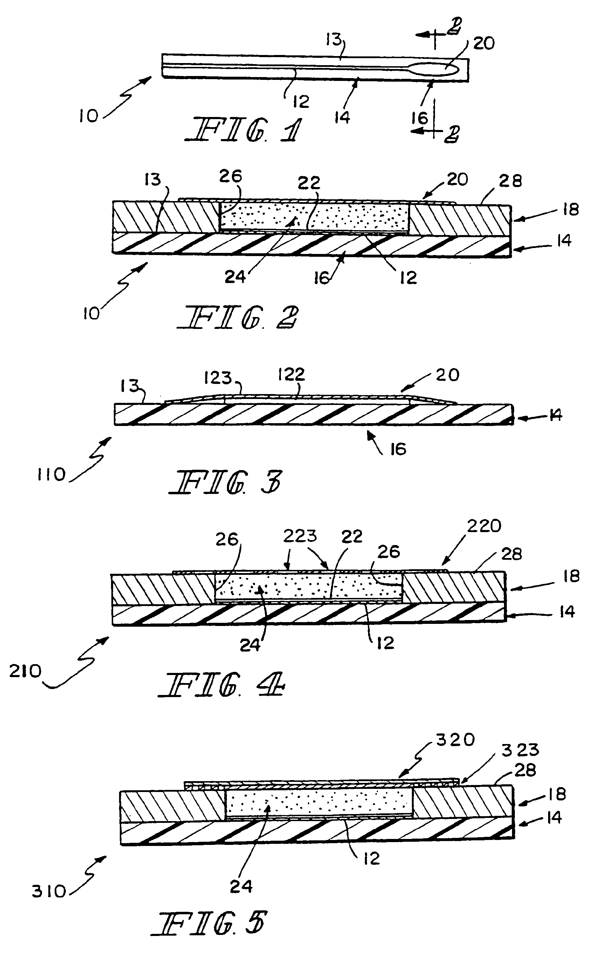

With reference to Fig. 1, there is provided in one embodiment of a sensor 10 utilizing a thin-film gold electrode 12 on an inert substrate 14. In cross section (see Fig. 2) a sensing portion 16 includes gold electrode 12 on a surface 13 of inert substrate 14, spacer layers 18 and a diffusion-limiting membrane 20 having small or no pores. Membrane 20 can be formed as a separate sheet and applied to spacer layers 18 resting on gold electrode 12 and inert substrate 14 to complete definition of an enclosure for a depletion volume 22 filled with a hydrophilic medium 24. The enzyme and mediator may be immobilized, for example by entrapment in a hydrophilic matrix on the gold electrode 12. Additionally, it is contemplated that the enzyme and mediator may be immobilized by covalent bonding to a wall 26 of enclosure 22 or to overlying membrane 20 or such components can be freely diffusing and selected to have minimal membrane permeability. Diffusion-limiting membrane 20 can be selected from art-recognized analyte permeable membranes and adhered to a surface 28 of spacer layer 18 to complete enclosure 22 for the analyte retention/depletion volume. Alternatively the analyte permeable membrane can be formed in situ by cross-linking polyfunctional hydrophilic polymer and/or monomer components of the enzyme/mediator reagent layer. It is understood that as used throughout the disclosure, that like reference numerals are used to denote like components.

With reference to Fig. 3, sensor 110 is provided in accordance with this disclosure Sensor 110 includes an electrode formed of a porous/particulate carbon layer wherein the depletion volume is defined by the interstitial spaces between carbon/graphite particles, (not shown). Thus a carbon-enzyme-mediator reagent layer 122 can be formed, for example by screen printing, a carbon/graphite suspension comprising a redox enzyme, and a nondiffusible or poorly diffusible, for example a polymeric, electron mediator in combination with one or more optionally non-electron mediating, polyfunctional polymers. The suspension is typically deposited on a conductor element (not shown) on an inert substrate. The diffusion-limiting analyte permeable membrane 20 can be applied, similar to that mentioned above, as a preformed polymer sheet applied and sealed over a surface 123 of the carbon electrode or membrane 20 can be formed by coating the exposed printed electrode having an intraparticulate polyfunctional polymer matrix with a polymer in solution. See, for example, International Patent Application No. WO 98/17995 for non-limiting examples of polymer membranes for coating biosensors.

With reference to Fig. 4, which is similar to Fig. 1, sensor *210* includes a diffusion-limiting membrane 220 that is formed to have large pores 223 for enhanced glucose diffusion. The mediator and enzyme components are preferably immobilized by entrapment in a hydrophilic matrix on electrode 12, by covalent bonding to nondiffusible polymeric components of hydrophilic medium 24, or to surfaces 26 of sensor enclosure 22 for the retention volume.

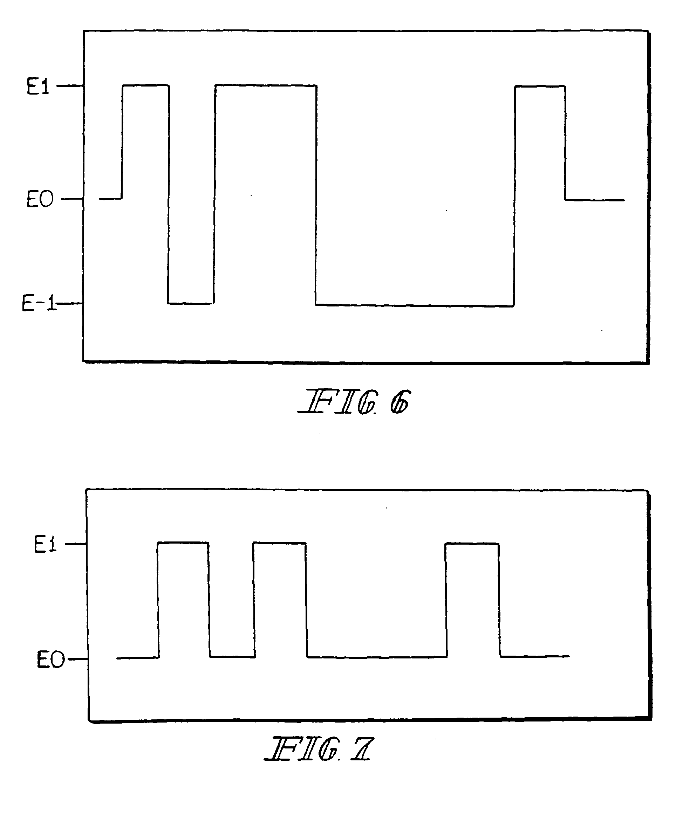

With reference to Fig. 5, there is provided another embodiment in the disclosure Sensor 310 is similar to that illustrated in Figs. 1 and 4 with the exception that membrane 320 overlying hydrophilic medium 24 is nonpermeable itself. Membrane 320 is provided with peripheral porosity allowing diffusion of glucose or other analytes into the retention/depletion volume.

The biochemical sensor can be constructed in any form adapted for the intended use. Thus, sensors intended for repeated laboratory use can be constructed in the form of an elongated probe having the sensor element itself located at one end and electrical conductors connecting the electrode component of the sensing element to points of electrical attachment of the probe sensor to a programmable sensor controller. Alternatively the present electrochemical sensor can be constructed using art recognized micro scale manufacturing techniques to produce needle-like sensors capable of being implanted or injected into a site for indwelling sensor applications.

The electrochemical sensor of this disclosure can be utilized for monitoring analyte concentrations in biological fluids. The method comprises the steps of contacting the biological fluid with the sensor and at initially predetermined intervals intermittently applying a potential to the electrode sufficient to oxidize the electron mediator. The current passing through the electrode is then sensed as a function of the duration of the applied potential. The applied mediator-oxidizing potential is maintained for at least a period of time sufficient to determine the rate of change of current with time through said electrode. The sensed current flow is then correlated with current flow for known concentrations of said analyte in the retention/detection medium. Alternatively the sensor can be constructed to comprise at least a working electrode and a reference electrode and optionally an auxiliary electrode, which may be identical with the referenced electrode. At initially predetermined intervals a potential is applied sufficient to establish a predetermined level of current flow between the working electrode and the auxiliary electrode. And the potential difference between the working and reference electrodes necessary to establish said level of current flow is measured and maintained for a period of time sufficient to determine the rate of change of potential necessary to maintain said current flow through said electrode. The potential measurements are then correlated with potential measurements recorded for known concentrations of analyte in the biological fluid.

The applied potentials, the duration of the potential pulses, and the intervals between potential pulses are entered into a programmable controller used in conjunction with the sensor for analyte measurements. In one embodiment the intervals between the intermittent applied potentials are less than the time necessary for the concentration of the analyte in the retention volume to equilibrate with that in the biological fluid in contact with the analyte permeable membrane. In another embodiment the intervals are increased incrementally for a series of applied potentials, and the concentration of the analyte and the biological fluid is determined as a function of the rate of increase in analyte concentration in the retention volume. The intervals between applied potential pulses can be modified based on previous measurements to adjust for variations in sensor performance deriving from loss or degradation of redox enzyme and electron mediator components and/or change in diffusion characteristics of either the analyte permeable membrane or the retention volume medium. Alternatively the duration of the applied potential pulse is modified based on previous measurements of sensed sensor performance status. In still another embodiment of sensor operation the intervals between measurements is substantially equal to or greater than the time required for the analyte concentration in the retention volume to equilibrate with that in the biological fluid. The method according to this invention is specified in the appended claims.

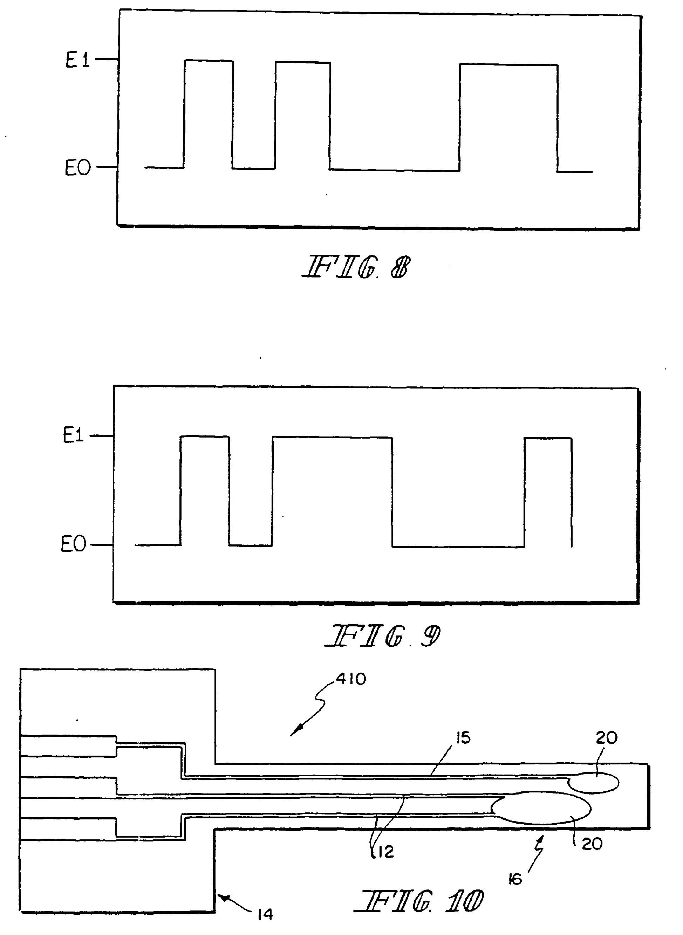

Figs. 6-9 present graphic illustrations of sample measurement algorithms wherein the potential of the electrode is controlled over a period of time to vary between a potential at which no oxidation of mediator occurs (E₀) to a mediator oxidizing potential (E1) and a potential at which reduction of the mediator takes place (E-1). The potential protocol to be applied in any given situation depends on sensor status, the form of the sensor, and the nature of the electron mediator and redox enzyme. The protocol for sensor operation can be optimized by empirical measurement and observations by the skilled user.

Specifically, Fig. 6 shows a pulse sequence in which two pulses of oxidative potential of different duration are applied to the sensor, interspersed with recovery intervals with reducing potential. By comparing the current profile from the first pulse with that from the second, information on the rate of enzymatic turnover of the substrate, and the rate of electron diffusion within the sensor may be obtained. Between the oxidative potential applications, the reducing potential ensures that all of the mediator is returned to it's initial state prior to the application of the next oxidizing potential.

Fig. 7 shows a sequence in which the duration of the intervals between the pulses is changed. By comparing the current observed from the second pulse to that from the first, information on the recovery time of the sensor can be gained. The recovery time yields information not only on analyte concentration, but also on diffusion into the hydrophilic matrix. Measuring the rest potential of the sensor between the potentiostatic pulses also provides information on the sensor recovery.

Figs. 8-9 demonstrate measurement protocols, which combine changes in the pulse interval with changes in the pulse width. The controller can select from a variety of sequences and durations of amperometric measurement intervals and recovery intervals to determine not only the analyte concentration, but also probe the condition of the sensor for enzyme activity, diffusion of substrate and mediator within the sensor, and diffusion of substrate into the sensor.

The nature and construction of the controller used with the present electrochemical sensor is not critical provided that the controller can be programmed to apply the appropriate potential pulse profiles to the sensor electrode or electrode system and to sense the current flow as a function of potential in time. A separate sensor status assessment protocol can be implemented in which the controller implements a protocol that allows computation of diffusion characteristics of the depletion volume and membrane from current data. The controller can be programmed to adjust measurement intervals and pulse widths based on computed diffusion times and determined substrate concentration. Both chronoamperometry and chronocolometry may be used to determine sensor status and substrate concentration. Ideally the controller should also be capable of applying a reducing potential to the electrode to reduce the mediator and thereby decrease consumption of analyte between measurements.

### EXAMPLE 1:

A sensor is formed similarly to sensor 10 of Figs. 1-2, except that it does not include membrane 20. The enzyme and electron mediator is entrapped in a polymer matrix on the electrode. This polymer matrix was prepared by electropolymerization of pyrrole and pyrrole-mediator derivatives. This method entrapped the enzyme in a polymer matrix on the electrode, and, by incorporating mediator-derivalized pyrrole into the polymer, provided for an immobilized mediator for transfer of electrons within the sensor.

Platinum disc electrodes were suitable for preparing sensors in accordance with this method. A mediator which was suitable for copolymerization in a matrix was be prepared by the following reaction sequence:

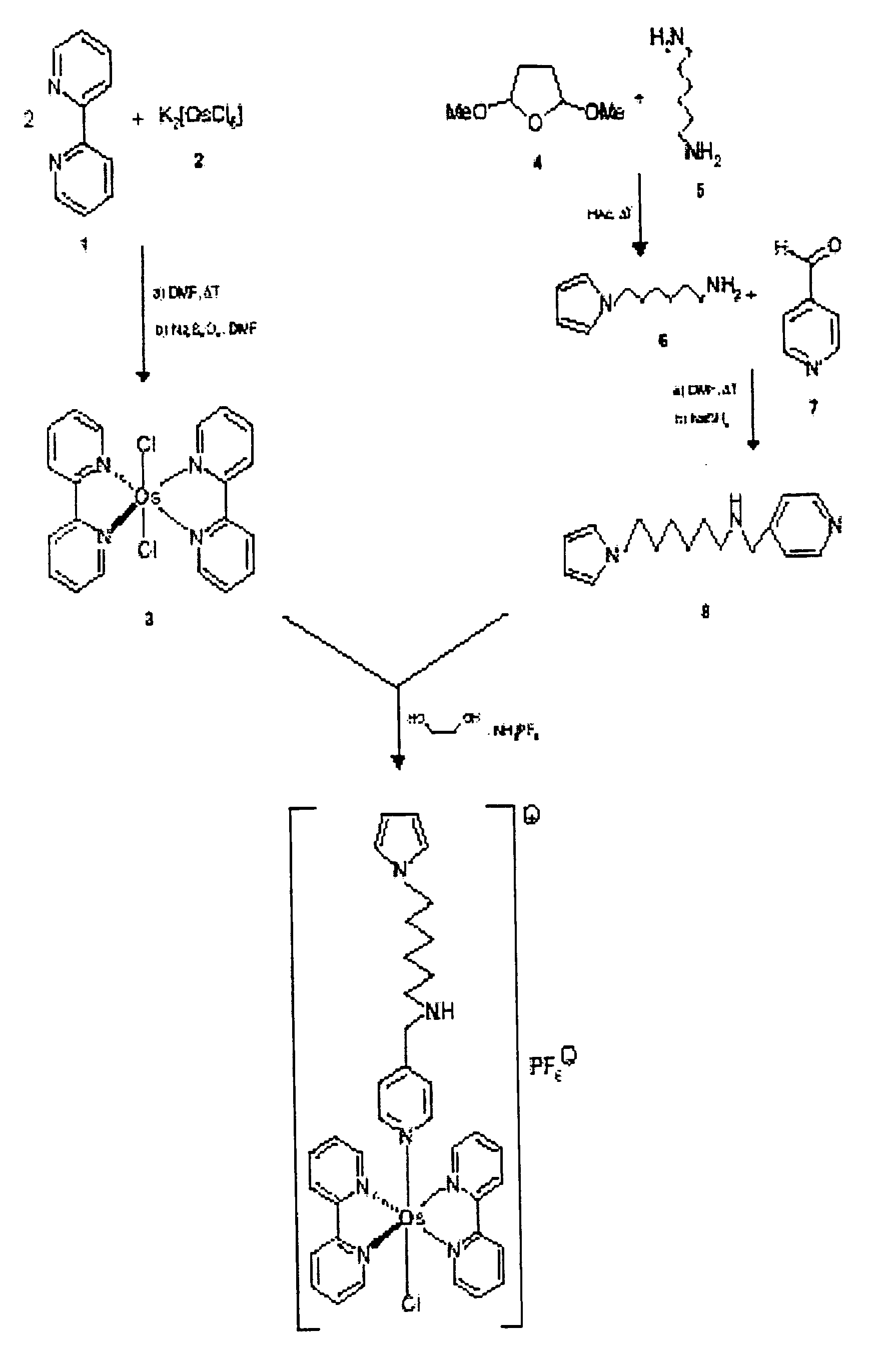

### Synthesis Of Pyrrole-Modified Osmium (Bisbipyridyl) Pyridinium Chloride

Pyrrole-modified Osmium (bisbipyridyl) pyridinium Chloride was prepared by the following reaction sequence:

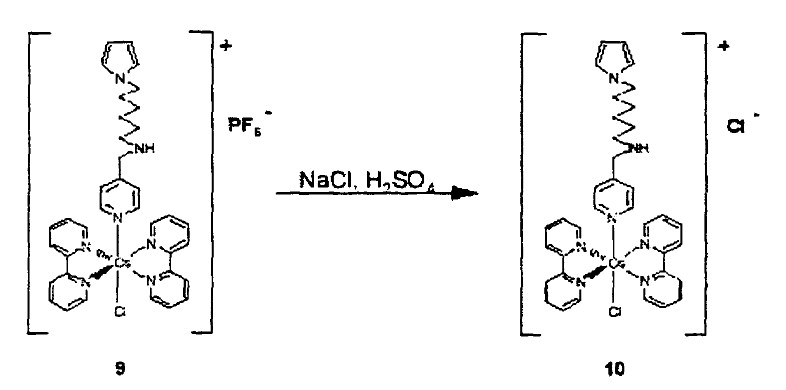

### Cleaning Procedure For Platinum Electrodes

The platinum disc electrodes were polished with Al₂O₃-paste of decreasing roughness (3µ m, 1µm, 0.1µm) on a polishing cloth and were washed with distilled water. Next, the electrodes were cleaned in an ultrasonic bath first in 10 M NaOH and then in 5 M H₂SO₄- ten minutes each.

The electrodes underwent electrochemical cycling in oxygen-free 0.5 M H₂SO₄:
1. Scan: -610 to + 1000 mV vs. Hg/HgSO₄ (100 mV/s, 10µA)
2. Scan: -810 to + 1600 mV vs. Hg/HgSO₄ (100 mV/s, 10µA)
3. Scan: -610 to + 1000 mV vs. Hg/HgSO₄ (100 mV/s, 10µA)
The third scan was repeated until the cyclic voltagram showed a clean platinum surface and stayed constant.

The last step was the polarization at -210 mV for five minutes.

### Platinization

Additionally, 4 ml of an oxygen-free 2 mM H₂PtCl₆-solution was prepared under Argon in a degassed electrochemical cell. Three voltammetric cycles from +500 to -400 mV vs. Ag/AgCl with a scan rate of 10 mV/s were applied. Then the electrode was washed under argon with oxygen-free distilled water and stored under argon until use.

### Polymerization Of Pyrrole/Mediator

A pyrrole film was polymerized to contain active Osmium (bis-bipyridyl) pyridinium Chloride as follows:
Co-polymerization was carried out with a mixture of 2mM pyrrole, 8mM (9) and 25 mM Tetramethyl ammonium perchlorate (as electrolyte) in 1:1 mixture of acetonitrile: water. 100 Pulses with potential / time of 0 V for sec / 1.5 V 1 sec. were carried out. The polymerization was carried out in the absence of oxygen.

### Polymerization Of Pyrrole-3-Acetic Acid/ GDH

In a solution of 50 mM Pyrrole-3-acetic acid, 0.05 mM PQQ, and 50 mM KCl in 0.1 M HEPES buffer, 5 mg/mL of GDH was dissolved. Following a 30 minute incubation to allow for reconstitution of the apoenzyme, the solution was polymerized with potential pulse profile consisting of 20 pulses of 0 V. for 5 sec /1.2 V for 1 second.

### Polymerization Of Pyrrole-3-Carboxylic Acid/ GDH

In a solution of 50 mM Pyrrole-3-carboxylic acid and 50 mM KCl in 0.1 M Hepes buffer, 5 mg/mL of sGDH was dissolved. The solution was polymerized with potential pulse profile consisting of 20 pulses of 0 V. for 5 sec / 1.2 V for 1 second, or alternatively of 0 V. for 5 sec / 1.4 V for 1 second, or also of 0 V. for 5 sec / 1.6 V for I second. Potential impacted the sensor performance considerably, indicating the dependence on polypyrrole film properties.

### Polymerization Of Pyrrole-3-Acetic Acid/Mediator/ GDH

In a solution of 50 mM Pyrrole-3-acetic acid, 10 mM Osmium modified pyrrole derivative 10, 0.05 mM PQQ and 50 mM KCl in 0.1 M HEPES buffer 5 mg/mL of sGDH was dissolved. The solution was polymerized with potential pulse profile consisting of 20 pulses of 0 V. for 5 sec / 1.4 V for 1 second.

### Polymerization Of Pyrrole-3-Carboxylic Acid/Mediator/ GDH

In a solution of 50 mM Pyrrole-3-acetic acid, 10 mM Osmium modified pyrrole derivative 10, 0.05 mM PQQ and 50 mM KCl in 0.1 M HEPES buffer 5 mg/mL of sGDH was dissolved. The solution was polymerized with potential pulse profile consisting of 20 pulses of 0 V. for 5 sec / 1.4 V for 1 second.

Enzyme electrodes were washed with deionized water. It is appreciated, however, that the enzyme electrodes may be washed with a buffer solution or, if necessary, with 3M KCl solution to remove adsorbed GDH and afterwards stored in 0.1 M phosphate buffer pH 7. If electrodes are stored over-night they are kept at 4°C otherwise at RT.

### Evaluation Of Sensor Response To Glucose

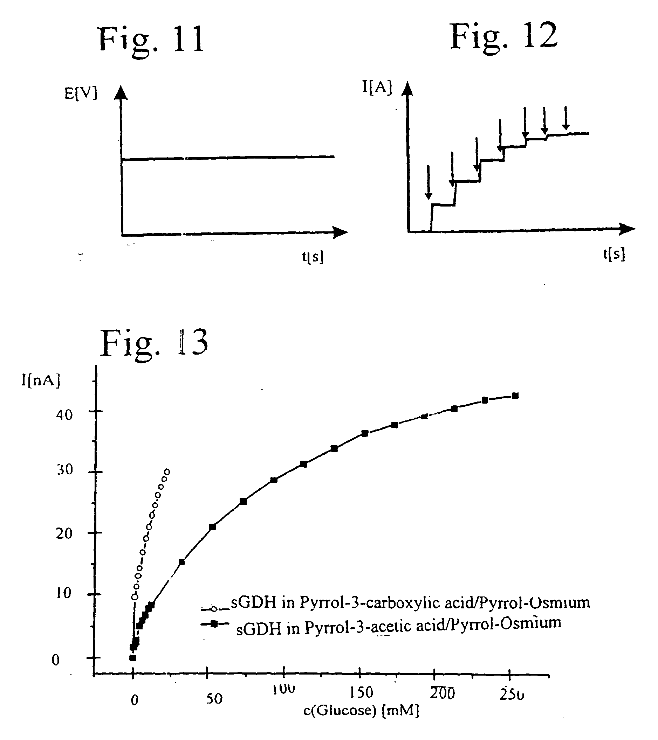

Sensor response was measured by placing the sensor into a stirred PBS (Phosphate buffered saline) buffer along with a reference electrode and a counter electrode. A potential (E) sufficient to oxidize the immobilized mediator was applied, and the solution stirred for a time (t) until the current (1) stabilized to a low value. See Fig. 11. Aliquots of 1M glucose solution in PBS were added in a step-wise manner, as shown by arrows in Fig. 12, to the PBS buffer to increase the concentration of glucose in the stirred PBS buffer solution, and the current (1) was measured on the plateau region of the response curve.

Referring now to Fig. 13, the response was measured for the Pyrrole-3-acetic acid/Mediator/GDH and pyrrole-3-carboxylic acid/mediator/GDH sensors. The response shows good electron transfer through the immobilized mediator in the polypyrrole film.

### EXAMPLE 2:

A sensor 410 consists of conductors 12, 15 and reagents deposited on a flat polymeric substrate 14. Materials for encapsulation of the conductors 12, 15 are provided, and reagents that form a semipermeable biocompatible layer over the reagent-containing sensing area 16. See Fig. 10.

### Processes and materials:

- Substrate:: Polyimid (such as Kapton^{®} polyimide film, which is commercially available from E.I. DuPont de Nemours, Wilmington, Delaware, and Upilex^{®} polimide film which is commercially available from UBE Industries Ltd, Japan) 0.005" (0.127 mm) thick with gold electrodes and conducting tracks.
- Processing:: Material is washed with water, acetone, and methylene chloride, then dried at 180° C for 20 hours. Material is placed in a vacuum chamber and metalized with 50 *Å* Chromium followed by 500 *Å* Gold. Metalized material is removed. A laminated photoresist is applied. The resist is exposed, and developed in an aqueous salt solution. Then the metal pattern is developed in a metal etchent solution (typically HNO₃_HCl). The patterned material is rinsed with water, and the remaining photomask is removed with a solvent (typically N-Methylpyrrolidone (NMP). A photodefinable polyimid layer 2 um thick is applied with a spin coater, and solidified by baking at 80° C for 20 minutes. It is exposed and then developed with a solvent (NMP). It is then baked at 200° C for 30 minutes to harden the remaining polymer.
- Reference/ Counter:: A silver/silver chloride ink preparation consisting of suspension of silver particles, partially converted to silver chloride on their surfaces, in an organic solvent, i.e. cyclohexanone, with a polymeric suspending agent, such as alginate.
- Processing:: The ink mixture is applied to the opening in the polyimid and dried for 30 minutes at 80° C to remove all solvent.
- Sensor Area:: The opening through the polyimid to the gold electrode is covered with a multilayer reagent area consisting of the following reagents.

### Active

- Enzyme Layer:: A solution of Glucose Dehydrogenase/ pyrroloquinoline quinone (PQQ) (Enzyme Commission No. 1.1.99.17) and a redox polymer consisting of polyvinylimidazole with bis (bipyridyl) chloro-Osmium in phosphate buffer, optionally with a polyhydric synthetic, natural or semisynthetic polymer, is applied to one electrode opening and dried. A solution of polyethylene glycol diglycidyl ether in aqueous buffer (i.e. 10 mM NaPO4, 150 mM NaCl) is applied to the area, and allowed to dry at room termperature overnight. The electrodes are then rinsed in physiological saline solution and allowed to dry.

### Biocompatibility

- Layer:: The entire structure is placed in a vacuum chamber. A plasma of diglyme is created in the chamber by introducing a low pressure vapor, and applying an RF field to dissociate the diglyme and cause polymerization. After 5 minutes, the diglyme addition is stopped, and the plasma continued for a further 5 minutes. Then the vacuum is broken and the sheet removed from the chamber.
- Post-Processing:: The sensors 410 are punched from the sheet and mounted into small polycarbonate holders which protect the sensing area and serve as the insertion device. The packaged sensors are sealed into polyethylene bags. The bagged sensors are radiation-sterilized. Ten sterilized sensors are packed into a larger plastic box containing desiccant in the lid. The box is closed and packed into a cardboard outer pack that serves as the final consumer package.

This method for the fabrication of sensors suitable for use according to the current disclosure utilizes the so-called "wired-enzyme" technology for the immobilization of enzyme and mediator in active relationship within a sensor film. The GDH/PQQ substantially does not react with any endogenous substrate other than the analyte of interest. This allows the sensor to remain inactive, or "off'' so long as the electrode is not regenerating the mediator to allow further activity. Sensors produced by this method demonstrate very high sensitivity to glucose and very high current density.

## Claims

1. A method for monitoring analyte concentration in a biological fluid by use of a sensor, the method comprising the steps of:
providing a sensor comprising a retention volume of a hydrophilic medium, the retention volume being formed to retain an amount of analyte that is proportionate to the concentration of the analyte in a biological fluid in contact with the sensor, an enclosure for said analyte retention volume, the enclosure being defined at least in part by a wall comprising an area of an analyte permeable membrane (20, 220) having a first side in contact with the hydrophilic medium, an electrode in contact with the hydrophilic medium, a redox enzyme in contact with the medium, and an electron transfer mediator for facilitating transfer of electrons between the enzyme and the electrode, the enzyme substantially not capable of transferring electrons to or from components of the biological fluid other than said analyte,
contacting the biological fluid with the sensor and at intervals applying a potential to the electrode sufficient to oxidize the electron mediator and sensing current through said electrode as a function of the duration of the applied potential,
maintaining the applied mediator-oxidizing potential at least for a period of time sufficient to determine the rate of change of current with time through the electrode, and
correlating the current flow with the current flow for known concentrations of said analyte in the retention medium
wherein the method further comprises the step of applying a potential to the electrode sufficient to reduce the oxidized form of the electron mediator.

2. The method of claim 1 wherein the intervals between the applied potentials are less than the time necessary for the concentration of the analyte in the retention volume to equilibrate with that in the biological fluids in contact with the enclosure.

3. The method of claim 2 wherein the electron mediator is osmium (bis-bipyridyl) pyridinium chloride.

4. The method of claim 2 wherein the redox enzyme and electron mediator are entrapped in a hydrophilic matrix on the electrode.

5. The method of claim 1 wherein the intervals are increased incrementally for a series of applied potentials and the concentration of the analyte in the biological fluid is determined as a function of the rate of increase in analyte concentration in the retention volume.

6. The method of claim 5 wherein the intervals between applied potential pulses are modified based on previous measurement results.

7. The method of claim 5 wherein the duration of the applied potential pulse is modified based on previous measurement results.

8. The method of claim 1 wherein the interval between the applied potentials is substantially equal to or greater than the time required for the analyte concentration in the retention volume to equilibrate with that in the biological fluid.

9. The method of claim 1, 2 or 8 wherein the analyte is glucose and the redox enzyme is a glucose dehydrogenase.

10. The method of claim 1, 2 or 8 wherein the analyte is glucose and the redox enzyme is a pyrroloquinoline quinone (PQQ)-dependent glucose dehydrogenase

11. The method of claim 1, 2, 5, 6, 7, or 8, wherein the sensor additionally comprises at least a reference electrode and an auxiliary electrode and further comprising the steps of intermittently establishing predetermined level of current to flow between said working electrode and said auxiliary electrode at initially predetermined intervals, and measuring the potential difference between said working electrode and the reference electrode; maintaining said level of current flow at least for a period of time sufficient to determine the rate of change of potential necessary to maintain said current through said electrode with time, and correlating said potential with the potential for known concentrations of said analyte in the biological fluid.

12. The method claim 1 further comprising a programmable controller in electrical contact with the electrode to control the electrical potentials at said electrode and sensing current through said electrode responsive thereto.

13. The method of claim 1, wherein 2 pulses of oxidative potential of different duration are applied to the sensor interspersed with recovery intervals with reducing potential.

14. The method of claim 1, wherein the potential of the electrode is controlled over a period of time to vary between a potential at which no oxidation of mediator occurs to a mediator oxidising potential and a potential at which reduction of the mediator takes place.

15. The method of claim 1, wherein between the oxidative potential applications, the reducing potential ensures that all of the mediator is returned to its initial state prior to application of a next oxidizing potential.

16. The method of claim 1, further comprising the step of applying a next oxdizing potential after applying the potential sufficient to reduce the oxidized form of the electron mediator.

## Patentansprüche

1. Verfahren zur Überwachung der Analytenkonzentration in einer biologischen Flüssigkeit durch Verwendung eines Sensors, wobei das Verfahren die folgenden Schritte umfasst:
Bereitstellen eines Sensors mit einem Retentionsvolumen eines hydrophilen Mediums, wobei das Retentionsvolumen gebildet wird, um eine Analytenmenge zu halten, die der Konzentration des Analyten in einer biologischen Flüssigkeit, die mit dem Sensor in Berührung steht, proportional ist, einem Gehäuse für das Analyten-Retentionsvolumen, wobei das Gehäuse zumindest teilweise von einer Wand definiert wird, die einen Bereich aus einer für Analyten durchlässigen Membran (20, 220) mit einer ersten Seite, die mit dem hydrophilen Medium in Berührung ist, eine Elektrode, die mit dem hydrophilen Medium in Berührung ist, ein Redoxenzym, das mit dem Medium in Berührung ist und einen Elektronentransfervermittler zur Erleichterung des Elektronentransfers zwischen dem Enzym und der Elektrode aufweist, wobei das Enzym im Wesentlichen nicht fähig ist, Elektronen zu oder von Komponenten der biologischen Flüssigkeit außer dem Analyten zu übertragen,
Inberührungbringen der biologischen Flüssigkeit mit dem Sensor und intervallmäßiges Anlegen eines Potentials an die Elektrode, das ausreicht, um den Elektronenvermittler zu oxidieren, und Messen des Stroms durch die Elektrode in Abhängigkeit von der Dauer des angelegten Potentials,
Aufrechterhalten des angelegten vermittler-oxidierenden Potentials zumindest über einen Zeitraum, der ausreicht, um die Stromwechselrate im Zeitverlauf durch die Elektrode zu bestimmen, und
Korrelieren des Stromflusses mit dem Stromfluss für bekannte Konzentrationen des Analyten im Retentionsmedium,
wobei das Verfahren ferner den Schritt des Anlegens eines Potentials an die Elektrode umfasst, das ausreicht, um die oxidierte Form des Elektronenvermittlers zu reduzieren.

2. Verfahren nach Anspruch 1, wobei die Intervalle zwischen den angelegten Potentialen kleiner sind als die Zeit, die notwendig ist, damit die Konzentration des Analyten im Retentionsvolumen mit der in den biologischen Flüssigkeiten, die mit dem Gehäuse in Berührung sind, äquilibriert.

3. Verfahren nach Anspruch 2, wobei der Elektronenvermittler Osmium (bispyridyl)pyridiniumchlorid ist.

4. Verfahren nach Anspruch 2, wobei das Redoxenzym und der Elektronenvermittler in einer hydrophilen Matrix auf der Elektrode eingeschlossen sind.

5. Verfahren nach Anspruch 1, wobei die Intervalle inkremental für eine Reihe von angelegten Potentialen erhöht werden und die Konzentration des Analyten in der biologischen Flüssigkeit in Abhängigkeit von der Anstiegsrate der Analytenkonzentration im Retentionsvolumen bestimmt wird.

6. Verfahren nach Anspruch 5, wobei die Intervalle zwischen den angelegten Potentialpulsen auf Basis der vorherigen Messergebnisse modifiziert werden.

7. Verfahren nach Anspruch 5, wobei die Dauer des angelegten Potentialpulses auf Basis der vorherigen Messergebnisse modifiziert wird.

8. Verfahren nach Anspruch 1, wobei das Intervall zwischen den angelegten Potentialen im Wesentlichen gleich oder größer ist als die Zeit, die notwendig ist, damit die Konzentration des Analyten im Retentionsvolumen mit der in der biologischen Flüssigkeit äquilibriert.

9. Verfahren nach Anspruch 1, 2 oder 8, wobei der Analyt Glucose ist und das Redoxenzym Glucosedehydrogenase ist.

10. Verfahren nach Anspruch 1, 2 oder 8, wobei der Analyt Glucose ist und das Redoxenzym eine pyrrolochinolin-chinon(PQQ)-abhängige Glucosedehydrogenase ist.

11. Verfahren nach Anspruch 1, 2, 5, 6, 7 oder 8, wobei der Sensor zusätzlich zumindest eine Referenzelektrode und eine Hilfselektrode umfasst und ferner die Schritte der intermittierenden Bestimmung einer vorbestimmten Strommenge, die zwischen der Arbeitselektrode und der Hilfselektrode in anfänglich vorbestimmten Intervallen fließen soll, und der Messung des Potentialgefälles zwischen der Arbeitselektrode und der Referenzelektrode; Aufrechterhaltung der Stromflussmenge zumindest über einen Zeitraum, der ausreicht, um die Potentialwechselrate, die notwendig ist, um den Strom durch die Elektrode im Zeitverlauf aufrechtzuerhalten, zu bestimmen, und Korrelation des Potentials mit dem Potential für bekannte Konzentrationen des Analyten in der biologischen Flüssigkeit.

12. Verfahren nach Anspruch 1, ferner umfassend einen programmierbaren Regler in elektrischem Kontakt mit der Elektrode zur Kontrolle der elektrischen Potentiale an der Elektrode und Messen des Stroms durch die darauf reagierende Elektrode.

13. Verfahren nach Anspruch 1, wobei 2 Pulse des oxidativen Potentials unterschiedlicher Dauer mit dazwischenliegenden Erholungsintervallen mit reduziertem Potential an den Sensor angelegt werden.

14. Verfahren nach Anspruch 1, wobei das Potential der Elektrode über einen Zeitraum so kontrolliert wird, dass es zwischen einem Potential, bei dem keine Oxidation des Vermittlers zu einem vermittler-oxidierenden Potential auftritt, und einem Potential, bei dem es zur Reduzierung des Vermittlers kommt, schwankt.

15. Verfahren nach Anspruch 1, wobei zwischen dem Anlegen des oxidativen Potentials das reduzierende Potential dafür sorgt, dass der gesamte Vermittler vor Anlegen des nächsten oxidierenden Potentials wieder in seinen Ausgangszustand gebracht wird.

16. Verfahren nach Anspruch 1, ferner umfassend den Schritt des Anlegens eines nächsten oxidierenden Potentials nach dem Anlegen des Potentials, das ausreicht, um die oxidierte Form des Elektronenvermittlers zu reduzieren.

## Revendications

1. Procédé pour suivre la concentration en analyte dans un fluide biologique par l'utilisation d'un détecteur, le procédé comprenant les étapes :
- de mise à disposition d'un détecteur comprenant un volume de rétention d'un milieu hydrophile, le volume de rétention étant formé pour retenir une quantité d'analyte qui est proportionnelle à la concentration en analyte dans le fluide biologique en contact avec le détecteur, d'une enceinte pour ledit volume de rétention de l'analyte, l'enceinte étant définie, au moins en partie, par une paroi comprenant une zone d'une membrane perméable à l'analyte (20,220) présentant une première face en contact avec le milieu hydrophile, une électrode en contact avec le milieu hydrophile, une enzyme redox en contact avec le milieu et un médiateur de transfert d'électrons pour faciliter le transfert d'électrons entre l'enzyme et l'électrode, l'enzyme n'étant substantiellement pas capable de transférer des électrons vers ou à partir des composants du fluide biologique autres que ledit analyte,
- de mise en contact du fluide biologique avec le détecteur et, à des intervalles, d'application d'un potentiel à l'électrode, suffisant pour oxyder le médiateur d'électrons et de détection du courant dans ladite électrode en fonction de la durée du potentiel appliqué,
- de maintien du potentiel d'oxydation du médiateur appliqué pendant au moins un laps de temps suffisant pour déterminer la vitesse de changement du courant en fonction du temps dans l'électrode, et
- de corrélation du flux de courant avec le flux de courant de concentrations connues dudit analyte dans le milieu de rétention,
où le procédé comprend en outre l'étape d'application d'un potentiel à l'électrode suffisant pour réduire la forme oxydée du médiateur d'électrons.

2. Procédé selon la revendication 1 où les intervalles entre les potentiels appliqués sont inférieurs au temps nécessaire à la concentration en analyte dans le volume de rétention pour s'équilibrer avec celle dans les fluides biologiques en contact avec l'enceinte.

3. Procédé selon la revendication 2 où le médiateur d'électrons est le chlorure d'osmium-(bisbipyridyl)-pyridinium.

4. Procédé selon la revendication 2 où l'enzyme redox et le médiateur d'électrons sont piégés dans une matrice hydrophile sur l'électrode.

5. Procédé selon la revendication 1 où les intervalles sont augmentés par incréments pour une série de potentiels appliqués et la concentration en analyte dans le fluide biologique est déterminée en fonction de la vitesse d'augmentation de la concentration en analyte dans le volume de rétention.

6. Procédé selon la revendication 5 où les intervalles entre les impulsions de potentiel appliquées sont modifiés sur base de résultats de mesure antérieurs.

7. Procédé selon la revendication 5 où la durée de l'impulsion de potentiel appliquée est modifiée sur base de résultats de mesure antérieurs.

8. Procédé selon la revendication 1 où l'intervalle entre les potentiels appliqués est substantiellement égal à ou supérieur au temps nécessaire à la concentration en analyte dans le volume de rétention pour s'équilibrer avec celle dans le fluide biologique.

9. Procédé selon la revendication 1, 2 ou 8 où l'analyte est le glucose et l'enzyme redox est la glucose déshydrogénase.

10. Procédé selon la revendication 1, 2 ou 8 où l'analyte est le glucose et l'enzyme redox est la glucose déshydrogénase dépendant de la pyrroloquinoline quinone (PQQ).

11. Procédé selon la revendication 1, 2, 5, 6, 7, ou 8, où le détecteur comprend en outre au moins une électrode de référence et une électrode auxiliaire et comprend en outre les étapes d'établissement de manière intermittente du niveau de courant prédéterminé qui s'écoule entre ladite électrode de travail et ladite électrode auxiliaire à des intervalles initialement prédéterminés et de mesure de la différence de potentiel entre ladite électrode de travail et l'électrode de référence ; de maintien dudit niveau de flux de courant pendant au moins un laps de temps suffisant pour déterminer la vitesse de changement de potentiel nécessaire pour maintenir ledit courant dans ladite électrode en fonction du temps et de corrélation dudit potentiel avec le potentiel pour des concentrations connues dudit analyte dans le fluide biologique.

12. Procédé selon la revendication 1 comprenant en outre un contrôleur programmable en contact électrique avec l'électrode pour contrôler les potentiels électriques sur ladite électrode et la détection du courant dans ladite électrode en réponse à ceux-ci.

13. Procédé selon la revendication 1, où 2 impulsions de potentiel d'oxydation de durée différente sont appliquées au détecteur écartées d'intervalles de récupération avec un potentiel de réduction.

14. Procédé selon la revendication 1, où le potentiel de l'électrode est contrôlé sur un laps de temps de manière à varier entre un potentiel auquel il ne se produit pas d'oxydation du médiateur jusqu'à un potentiel d'oxydation du médiateur et un potentiel auquel il se produit une réduction du médiateur.

15. Procédé selon la revendication 1. dans lequel, entre les applications de potentiel d'oxydation, le potentiel de réduction assure que la totalité du médiateur est retourné dans un état initial avant l'application du potentiel d'oxydation suivant.

16. Procédé selon la revendication 1, comprenant en outre l'étape d'application d'un potentiel d'oxydation suivant après l'application du potentiel suffisant pour réduire la forme oxydée du médiateur d'électrons.
